# EUROPEAN PATENT APPLICATION

(11) **EP 3 824 856 A1**
(43) Date of publication of application: **26.05.2021**
(21) Application number: 19210513.8
(22) Date of filing: 21.11.2019
(51) Int. Cl.: A61F 13/505, A61F 13/64, A61F 13/49, A61F 13/42

(54) **ABSORBENT ARTICLE, A RE-USABLE PART AND A RESPECTIVE METHOD**

(71) Applicant: Ontex BV, 9255 Buggenhout (BE); Ontex Group NV, 9320 Erembodegem (BE)
(72) Inventor: WEBER, Ainas, 53474 Bad Neuenahr-Ahrweiler (DE); IDELSON, Alissa, 53359 Rheinbach (DE); HEIRMAN, Lisa, 9250 Waasmunster (BE); HELLMOLD, Jens, 59269 Beckum (DE)
(74) Representative: Macchetta, Andrea

(57) **Abstract**

The current invention concerns to an absorbent article (100) comprising a first part being a disposable part (10), and a second part being a re-usable part (20) wherein the re-usable part (20) is configured to removably hold the disposable part (10), and wherein the absorbent article (100) comprises an electronic system (22) configured to measure data related to a wearer of the absorbent article, and/or to the absorbent article and/or to ambient conditions in close proximity to the wearer of the absorbent article.

## Description

### TECHNICAL FIELD

The invention pertains to the technical field of absorbent hygiene products. In particular, the present invention relates to an absorbent article for absorbing body fluids and exudates, such as urine and fecal material. More particularly, the present invention relates to absorbent garments, such as disposable diapers, which are configured to collect and contain fecal material and avoid leakage.

More particularly, the invention pertains to absorbent articles, like a diaper, components thereof and a method for assembling the same, comprising a re-usable part and an electronic system.

### BACKGROUND

Disposable diapers conventionally include a chassis having a liquid permeable top sheet, a liquid impermeable back sheet and an absorbent structure sandwiched between the top sheet and back sheet. Moreover, the chassis includes a back sheet, top sheet, and preferably barrier leg cuffs, as well as leg elastics. The chassis has a front body panel which, in use, extends over the stomach and front hip area of the user, and a rear body panel which, in use, extends over the back and the rear hip area of the user. Each of the body panels has a waist portion such that, when the diaper is fastened around the waist of the user, the waist portions provide a continuous encirclement of the user. In order to fasten the diaper around the waist of a user, a fastening system comprising fastening tabs is commonly employed. Fastening tabs may be provided on side panels which extend from lateral side edges of the diaper chassis.

Inherent with the use of such absorbent articles, eventually one or more wetness or exudate events will occur and thus a need for change of the absorbent article arises. The generated waste and its corresponding impact on the environment is nowadays an issue that must be taken into consideration.

As a possibility to reduce the environmental footprint, absorbent articles (for example baby or adult diapers) comprising a re-usable part have been envisaged in an effort to decrease the amount of single-use material.

WO 2009/043101 A1 discloses a disposable nappy including a disposable nappy body portion and a separate belt to secure the nappy body to the person. The belt includes a number of Velcro-type fastener portions that attach to a cover stock material of the nappy. The cover stock material is of a material that includes fibres that can retain the Velcro-type fasteners. Before the article can be put on the wearer, the belt and the chassis are typically brought together and made to engage with each other, e.g. by means of said Velcro mechanical fasteners. Nevertheless, for the "hybrid" type of diapers disclosed in WO 2009/043101 A1 it has been encountered that the mechanical stability achieved by such Velcro fasteners is not sufficient, so that the belt can easily disengage from the chassis, which is detrimental for the fit of the absorbent article compared to a one-piece garment. Thus, there is the need to improve the structure and attachment of hybrid diapers in order to enhance their functionality.

Furthermore, absorbent articles possessing different types of detecting means are known, aiming to alert a user or caregiver to a change within the article (e.g. a soiling event). Such detecting means allow the user or caregiver to readily determine whether or not an absorbent article needs to be changed, without the need for close inspection or removal of the article. Numerous kinds of wetness indicators have been proposed to be used within absorbent articles. Many of those are based on electrical detection using conductive elements separated by a distance on any of the layers of the absorbent article. Safety issues, nonetheless, may still arise if the electrical components and related sensors may be in direct contact with a user, especially in the case of baby diapers.

This problem can be overcome by using electronic systems for wetness detection and for monitoring several ambient conditions in the proximity of a wearer, parameters of an absorbent article and parameters of the wearer, which are integrated into different parts of an absorbent article.

The present invention aims to resolve at least some of the problems mentioned above. Accordingly, it is the object of this invention to provide an absorbent article with a re-usable part and an electronic system, a re-usable part of an absorbent article, and a method for attaching said re-usable part to a disposable part.

### SUMMARY OF THE INVENTION

In view of the above-mentioned problems and disadvantages of prior art products, the object of the present invention is achieved by the solution provided in the enclosed independent claims. Advantageous implementations of the present invention are further defined in the dependent claims.

In a first aspect, the invention refers to an absorbent article comprising a first part being a disposable part, and a second part being a re-usable part, wherein the re-usable part is configured to removably hold the disposable part and wherein the absorbent article comprises an electronic system configured to measure data related to a wearer of the absorbent article, and/or the absorbent article and/or to ambient conditions in close proximity to the wearer of the absorbent article.

Advantageously, by having a re-usable part, the amount of single-use material is reduced. Further advantageously, this facilitates "design for recyclability" or "design for (bio)degradability", enabling to include in a disposable part less different types of materials than a conventional disposable article, optimizing the economical use of absorbent articles and reducing the amount of waste created. Moreover, the incorporation of an electronic system enables an accurate, automatic and safe detection of liquids and exudates and further body properties of a wearer, in an efficient manner.

According to a first preferred implementation form of the first aspect, the electronic system comprises at least one type of sensor and a transmitter, for transmitting measured data related to the absorbent article and/or to ambient conditions in close proximity to a wearer. This is beneficial, since wetness and ambient conditions associated to the wearer can be detected and further processed in order to determine wearer-related information, including risk of leakage within the absorbent article and relevant body parameters.

In a second preferred implementation form of the first aspect, the electronic system further comprises an energy harvester. Advantageously, energy to the different elements of the electronic system can be directly and automatically supplied.

According to a third preferred implementation form of the first aspect, the at least one type of sensor is a humidity sensor and/or a wetness sensor and/or a sensor for measuring ambient parameters and/or a sensor for measuring body parameters and/or a sensor for measuring parameters of the absorbent article and/or any combination thereof. This enables detecting and monitoring wetness and/or stool, which is particularly beneficial for babies with sensitive skin. Further advantageously, the invention enables to detect and observe various body parameters related to the wearer, increasing efficiency and functionality of the absorbent article.

In a further preferred implementation form of the first aspect, the electronic system or at least one element of the electronic system is i) included in a device separated from the re-usable part and the disposable part, in a manner that it is mechanically connected to the absorbent article; additionally or alternatively ii) integrated into the re-usable part, additionally or as an alternative iii) integrated into the disposable part and/or any combination thereof. Advantageously, the risk of a baby taking off and swallowing at least a part of the electronic system, for example a detachable sensor as proposed in the prior art, is overcome since it is not possible for a user to put the entire re-usable part inside the mouth. In addition to this safety aspect, the comfort for the wearer is improved by including the electronics in the form of smart textiles instead of using a rigid electronic system device.

According to a further preferred implementation form of the first aspect, the re-usable part is configured to extend at least along a width of the disposable part and to extend along at least a portion of a length of the disposable part. In addition to this or as an alternative, the disposable part comprises at least an absorbent core. This is beneficial, since fitting and functionality of the absorbent article, when worn by a user, is ensured. Furthermore, comfort for the wearer is also achieved.

In a further preferred implementation form of the first aspect, the re-usable part comprises an elongated part in the form of a belt, whereas the disposable part comprises a chassis of the absorbent article. Advantageously, this ensures both fitting of the absorbent article and comfort of the wearer when the article is worn. In addition to this, the re-usable part is less prone to be contaminated with body fluids, enhancing its lifetime.

According to a further preferred implementation form of the first aspect, the re-usable part comprises at least a portion of a chassis of the absorbent article whereas the disposable part comprises remaining portions of the chassis. This is beneficial, since it allows to design the absorbent article in order to optimize the type and amount of components that can be disposable.

In a further preferred implementation form of the first aspect, the re-usable part comprises a garment to be worn by a user and at least a portion of the absorbent article, whereas the disposable part comprises remaining portions of the chassis. Advantageously, this enables to reduce the amount of disposable material by integrating a disposable part of the absorbent article into a complete, wearable smart electronics garment.

According to a further preferred implementation form of the first aspect, the re-usable part comprises a fastening system of the absorbent article. Alternatively, the disposable part comprises a fastening system of the absorbent article, or as a further alternative the re-usable part comprises elements of the fastening system of the absorbent article and the disposable part comprises complementary elements of the fastening system. This is beneficial, since fitting, comfort and functionality of the absorbent article when worn by a user is ensured.

According to a further preferred implementation form of the first aspect, the absorbent article further comprises at least one holding means configured to attach the re-usable part to the disposable part. Additionally, the holding means is present on the disposable part or on the re-usable part or on the disposable part and the re-usable part. In this manner, proper functionality of the absorbent article as a whole is achieved by reinforcing the attachment between the re-usable and disposable parts, whilst ensuring proper electrical connection between elements of the electronic system.

In a second aspect, the present invention provides a re-usable part of an absorbent article configured to removably hold a disposable part of the absorbent article, wherein the re-usable part of the absorbent article comprises an electronic system, or at least one element of the electronic system, the electronic system being configured to measure data related to a wearer of the absorbent article, and/or the absorbent article and/or to ambient conditions in close proximity to the wearer of the absorbent article. Advantageously, providing such a re-usable part allows to reduce the amount of single-use material in an absorbent article. Further advantageously, this facilitates "design for recyclability" or "design for (bio)degradability", enabling to include in a disposable part less types of materials than a conventional disposable article, optimizing the economical use of said articles and reducing the amount of waste created. Moreover, the incorporation of an electronic system enables an accurate, automatic and safe detection and monitoring of liquids and exudates and further body properties of a wearer, in an efficient manner.

According to a first preferred implementation form of the second aspect, the electronic system comprises at least one type of sensor and a transmitter for transmitting the measured data related to the wearer of the absorbent article, and/or the absorbent article, and/or to ambient conditions in close proximity to the wearer of the absorbent article. In addition to this, the at least one type of sensor is a humidity sensor and/or a wetness sensor and/or a sensor for measuring ambient parameters and/or a sensor for measuring body parameters and/or a sensor for measuring parameters of the absorbent article and/or any combination thereof. This is beneficial, since wetness and ambient conditions associated to the wearer can be detected and further processed to determine wearer related information, including risk of leakage within the absorbent article and body parameters. Furthermore, this enables detecting and monitoring wetness and stool, which is particularly advantageous for babies with sensitive skin. In this manner, the invention enables to detect and observe various body parameters related to the wearer, increasing efficiency and functionality of the absorbent article.

Additionally, the electronic system preferably comprises an energy harvester. This allows for an independent wireless energy provision to the system.

In a second preferred implementation form of the second aspect, said re-usable part is configured to extend along at least a portion of a width of the disposable part and along at least a portion of a length of the disposable part. This is beneficial, since fitting and functionality of the absorbent article, when worn by a user, is ensured. Furthermore, the comfort for the wearer is also achieved.

According to a further preferred implementation form of the second aspect, said re-usable part is washable and/or is made of textile material. Additionally or alternatively, at least portions of said re-usable part have elastic properties. This is beneficial, since functionality, comfort and fitting of the re-usable part when attached to a disposable part forming the disposable article, are enhanced.

In yet a further aspect, the invention provides a method for holding parts of an absorbent article, comprising the steps of: providing a first part of the absorbent article being a disposable part; and providing a second part of the absorbent article being a re-usable part, wherein the re-usable part is configured to removably hold the disposable part; and wherein the method further comprises providing an electronic system, wherein the electronic system is configured to measure data related to a wearer of the absorbent article, and/or the absorbent article and/or to ambient conditions in close proximity to the wearer of the absorbent article. Advantageously, the method facilitates "design for recyclability" or "design for (bio)degradability", since the disposable part of the article is likely to include less types of materials than a conventional disposable article, optimizing the economical use of said articles and reducing the amount of waste created. Moreover, the incorporation of an electronic system enables an accurate, automatic and safe detection and monitoring of liquids and exudates and body parameters of a wearer in an efficient manner.

### DESCRIPTION OF FIGURES

**Figure 1** shows a first embodiment of an absorbent article according to the first aspect of the invention;
**Figure 2** shows a different view of an embodiment of the absorbent article according to the first aspect of the invention;
**Figure 3** illustrates a block diagram for an embodiment of an electronic system according to the invention;
**Figure 4** shows an exemplary embodiment of the absorbent article according to the first aspect of the invention, in which a wetness sensor comprises a conductive printed pattern;
**Figure 5A-B** shows an exemplary embodiment of the absorbent article according to the first aspect of the invention;
**Figure 6** illustrates a second embodiment of an absorbent article according to the first aspect of the invention;
**Figure 7** shows a third embodiment of an absorbent article according to the first aspect of the present invention;
**Figure 8** depicts a flow chart of a representative embodiment of the inventive method according to a further aspect of the invention.

### DETAILED DESCRIPTION OF THE INVENTION

The present invention concerns to an absorbent article with a re-usable part and an electronic system, a re-usable part of an absorbent article, and a method for attaching said re-usable part to a disposable part.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.

"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, in so far such variations are appropriate to perform in the disclosed invention. However, it is to be understood that the value to which the modifier "about" refers is itself also specifically disclosed.

"Comprise", "comprising", and "comprises" and "comprised of" as used herein are synonymous with "include", "including", "includes" or "contain", "containing", "contains" and are inclusive or open-ended terms that specifies the presence of what follows e.g. component and do not exclude or preclude the presence of additional, non-recited components, features, element, members, steps, known in the art or disclosed therein.

The expression "% by weight" (weight percent), here and throughout the description unless otherwise defined, refers to the relative weight of the respective component based on the overall weight of the formulation.

The recitation of numerical ranges by endpoints includes all numbers and fractions subsumed within that range, as well as the recited endpoints.

"Absorbent article" refers to devices that absorb and contain liquid, and more specifically, refers to devices that are placed against or in proximity to the body of the wearer to absorb and contain the various exudates discharged from the body. Absorbent articles include but are not limited to diapers, adult incontinence briefs, training pants, diaper holders and liners, sanitary napkins and the like, as well as surgical bandages and sponges. Absorbent articles preferably comprise a longitudinal axis and a transversal axis perpendicular to said longitudinal axis. The longitudinal axis is hereby conventionally chosen in the front-to-back direction of the article when referring to the article being worn, and the transversal axis is conventionally chosen in the left-to-right direction of the article when referring to the article being worn. Disposable absorbent articles can include a liquid pervious top sheet, a back sheet joined to the top sheet, and an absorbent core positioned and held between the top sheet and the back sheet. The top sheet is operatively permeable to the liquids that are intended to be held or stored by the absorbent article, and the back sheet may or may not be substantially impervious or otherwise operatively impermeable to the intended liquids. The absorbent article may also include other components, such as liquid wicking layers, liquid intake layers, liquid distribution layers, transfer layers, barrier layers, wrapping layers and the like, as well as combinations thereof. Disposable absorbent articles and the components thereof can operate to provide a body-facing surface and a garment-facing surface.

An absorbent article, such as a diaper, comprises a front waistband region, a back waistband region, an intermediate crotch region which interconnects the front and rear waistband regions. When used herein, reference to a "front" portion refers to that part of the diaper which is generally located on the front of an infant when in use. Reference to the "rear" portion refers to the portion of the diaper generally located at the rear of the infant when in use, and reference to the "crotch" portion refers to that portion which is generally located between the legs of an infant when in use. The crotch region is an area where repeated fluid surge typically occurs.

Preferably, a diaper comprises a liquid permeable "top sheet", a liquid impermeable "back sheet", and an "absorbent medium" disposed between the top sheet and the back sheet. The top sheet, back sheet and the absorbent medium could be made from any suitable material known to the person skilled in the art. The top sheet is generally located at or near the bodyside surface of the article, while the back sheet is generally located at or near the garment-side surface of the article. Optionally, the article may comprise one or more separate layers which are in addition to the back sheet and are interposed between the back sheet and the absorbent medium. Top sheet and back sheet are connected or otherwise associated together in an operable manner.

The "absorbent medium" or "absorbent core" or "absorbent body" is the absorbent structure disposed between the top sheet and the back sheet of the absorbent article in at least the crotch region of the absorbent article and is capable of absorbing and retaining liquid body exudates. The size and the absorbent capacity of the absorbent medium should be compatible with the size of the intended wearer and the liquid loading imparted by the intended use of the absorbent article. Further, the size and the absorbent capacity of the absorbent medium can be varied to accommodate wearers ranging from infants through adults. It may be manufactured in a wide variety of shapes (for example, rectangular, trapezoidal, T-shape, I-shape, hourglass shape, etc.) and from a wide variety of materials. Examples of commonly occurring absorbent materials are cellulosic fluff pulp, tissue layers, highly absorbent polymers (so called superabsorbent polymer particles (SAP)), absorbent foam materials, absorbent nonwoven materials or the like. It is common to combine cellulosic fluff pulp with superabsorbent polymers in an absorbent material.

"Acquisition and distribution layer", "ADL" or "surge management portion" refers to a sub-layer which preferably is a nonwoven wicking layer under the top sheet of an absorbent product, which speeds up the transport and improves distribution of fluids throughout the absorbent core. The surge management portion is typically less hydrophilic than the retention portion, and has the ability to quickly collect and temporarily hold liquid surges, and to transport the liquid from its initial entrance point to other parts of the absorbent structure, particularly the retention portion. This configuration can help prevent the liquid from pooling and collecting on the portion of the absorbent garment positioned against the wearer's skin, thereby reducing the feeling of wetness by the wearer. Preferably, the surge management portion is positioned between the top sheet and the retention portion.

The term "adhesive" as used herein is intended to refer to any suitable hot melt, water or solvent borne adhesive that can be applied to a surface of a film layer in the required pattern or network of adhesive areas to form the film-nonwoven laminate of the present invention. Accordingly, suitable adhesives include conventional hot melt adhesives, pressure-sensitive adhesives and reactive adhesives (i.e., polyurethane).

As used herein, the term "adhesive bonding" means a bonding process which forms a bond by application of an adhesive. Such application of adhesive may be by various processes such as slot coating, spray coating and other topical applications. Further, such adhesive may be applied within a product component and then exposed to pressure such that contact of a second product component with the adhesive containing product component forms an adhesive bond between the two components.

As used herein, an "airformed web" refers to a material comprising cellulosic fibers such as those from fluff pulp that have been separated, such as by a hammermilling process, and then deposited on a porous surface without a substantial quantity of binder fibers present. Airfelt materials used as the absorbent core in many diapers, for example, are a typical example of an airformed material.

As used herein, an "airlaid web" is a fibrous structure formed primarily by a process involving deposition of air-entrained fibers onto a mat, typically with binder fibers present, and typically followed by densification and thermal bonding. In addition to traditional thermally bonded airlaid structures (those formed with non-tacky binder material present and substantial thermally bonded), the scope of the term "airlaid" according to the present invention can also include coform, which is produced by combining air-entrained dry, dispersed cellulosic fibers with meltblown synthetic polymer fibers while the polymer fibers are still tacky. Further, an airformed web to which binder material is subsequently added can be considered within the scope of the term "airlaid" according to the present invention. Binder can be added to an airformed web in liquid form (e.g., an aqueous solution or a melt) by spray nozzles, direction injection or impregnation, vacuum drawing, foam impregnation, and so forth. Solid binder particles can also be added by mechanical or pneumatic means.

As used therein, the term "associated" encompasses configurations in which top sheet is directly joined to back sheet by affixing top sheet directly to back sheet, and configurations wherein top sheet is joined to back sheet by affixing top sheet to intermediate members which in turn are affixed to back sheet. Top sheet and back sheet can be affixed directly to each other by holding means such as an adhesive, sonic bonds, thermal bonds or any other holding means known in the art. For example, a uniform continuous layer of adhesive, a patterned layer of adhesive, a sprayed pattern of adhesive or an array of separate lines, swirls or spots of construction adhesive may be used to affix top sheet to back sheet. It should be readily appreciated that the above-described holding means may also be employed to interconnect and assemble together the various other component parts of the article described herein.

The terms "back section" and "rear back section" are used herein as synonyms and refer to the area of the absorbent article which is contact with the back of the wearer when the absorbent article is worn.

The term "back sheet" refers to a material forming the outer cover of the absorbent article. The back sheet prevents the exudates contained in the absorbent structure from wetting articles such as bedsheets and overgarments which contact the disposable absorbent article. The back sheet may be a unitary layer of material or may be a composite layer composed of multiple components assembled side-by-side or laminated. The back sheet may be the same or different in different parts of the absorbent article. At least in the area of the absorbent medium the back sheet comprises a liquid impervious material in the form of a thin plastic film, e.g. a polyethylene or polypropylene film, a nonwoven material coated with a liquid impervious material, a hydrophobic nonwoven material, which resists liquid penetration, or a laminate of a plastic film and a nonwoven material. The back sheet material may be breathable so as to allow vapour to escape from the absorbent material, while still preventing liquids from passing there through. Examples of breathable back sheet materials are porous polymeric films, nonwoven laminates of spunbond and meltblown layers and laminates of porous polymeric films and nonwoven materials.

The terms "belly section" and "front belly section" are used herein as synonyms and refer to the area of the absorbent article which is contact with the belly of the wearer when the absorbent article is worn.

The term "blend" means a mixture of two or more polymers while the term "alloy" means a sub-class of blends wherein the components are immiscible but have been compatibilized.

As used herein, the "body-facing" or "bodyside" surface means that surface of the article or component which is intended to be disposed toward or placed adjacent to the body of the wearer during ordinary use, while the "outward", "outward-facing" or "garment-side" surface is on the opposite side, and is intended to be disposed to face away from the wearer's body during ordinary use. Such outward surface may be arranged to face toward or placed adjacent to the wearer's undergarments when the absorbent article is worn.

"Bonded" refers to the joining, adhering, connecting, attaching, or the like, of at least two elements. Two elements will be considered to be bonded together when they are bonded directly to one another or indirectly to one another, such as when each is directly bonded to intermediate elements.

The term "breathable" refers to films having a water vapor transmission rate (WVTR) of at least 300 grams/m² - 24 hours.

"Carded web" refers to webs that are made from staple fibers that are sent through a combing or carding unit, which opens and aligns the staple fibers in the machine direction to form a generally machine direction-oriented fibrous nonwoven web. The web is then bonded by one or more of several known bonding methods. Bonding of nonwoven webs may be achieved by a number of methods; powder bonding, wherein a powdered adhesive or a binder is distributed through the web and then activated, usually by heating the web and adhesive with hot air; pattern bonding, wherein heated calendar rolls or ultrasonic bonding equipment are used to bond the fibers together, usually in a localized bond pattern, though the web can be bonded across its entire surface if so desired; through-air bonding, wherein air which is sufficiently hot to soften at least one component of the web is directed through the web; chemical bonding using, for example, latex adhesives that are deposited onto the web by, for example, spraying; and consolidation by mechanical methods such as needling and hydroentanglement.

As used herein, the term "cellulosic" is meant to include any material having cellulose as a major constituent, and specifically comprising at least 50 percent by weight cellulose or a cellulose derivative. Thus, the term includes cotton, typical wood pulps, nonwoody cellulosic fibers, cellulose acetate, cellulose triacetate, rayon, thermomechanical wood pulp, chemical wood pulp, debonded chemical wood pulp, milkweed, or bacterial cellulose.

"Chassis" refers to a foundational constituent of an absorbent article upon which the remainder of the structure of the article is built up or overlaid, e.g., in a diaper, the structural elements that give the diaper the form of briefs or pants when configured for wearing, such as a back sheet, a top sheet, or a combination of a top sheet and a back sheet.

"Coform" as used herein is intended to describe a blend of meltblown fibers and cellulose fibers that is formed by air forming a meltblown polymer material while simultaneously blowing air-suspended cellulose fibers into the stream of meltblown fibers. The coform material may also include other materials, such as superabsorbent particles. The meltblown fibers containing wood fibers are collected on a forming surface, such as provided by a foraminous belt. The forming surface may include a gas-pervious material, such as spunbonded fabric material, that has been placed onto the forming surface.

"Compression" refers to the process or result of pressing by applying force on an object, thereby increasing the density of the object.

The term "consisting essentially of" does not exclude the presence of additional materials which do not significantly affect the desired characteristics of a given composition or product. Exemplary materials of this sort would include, without limitation, pigments, antioxidants, stabilizers, surfactants, waxes, flow promoters, solvents, particulates and materials added to enhance processability of the composition.

The diaper can comprise "containment flaps" or "barrier cuffs". The containment flaps are generally thought to be particularly well suited for the containment of fecal matter and to prevent the lateral flow of liquid waste until such time as the liquid waste can be absorbed by the absorbent article. Many constructions of containment flaps are known. Such containment flaps generally comprise a proximal edge, intended to be attached to the absorbent article, and an opposite distal edge which is generally not attached to the absorbent article along at least a portion of its length. An elastic member is generally located adjacent the distal edge to assist in maintaining the containment flap in an upright condition and in maintaining a sealing relationship between the distal edge of the containment flap and the body of a wearer during use. The elastic member is generally located between two layers of material so that the elastic does not come into contact with the body of a wearer. The containment flaps may be manufactured from a wide variety of materials such as polypropylene, polyester, rayon, nylon, foams, plastic films, formed films, and elastic foams. A number of manufacturing techniques may be used to manufacture the containment flaps. For example, the containment flaps may be woven, non-woven, spunbonded, carded, cast, blown or the like.

The diaper can comprise leg containment gaskets. Leg "containment gaskets" help prevent leakage of bodily exudates when the wearer exerts compressive forces on the absorbent article. In particular, the stiffness of the leg containment gaskets prevents twisting and bunching of the leg openings of the absorbent article which can lead to leaks. In addition, the elasticity and conformability of the leg containment gaskets ensures that the bodyfacing surface of the leg containment gaskets provides an adequate seal against the body of the wearer. The physical properties of the leg containment gaskets, such as the thickness and stiffness, also function to space the bodyside liner, outer cover and absorbent core away from the wearer's body when in use. As such, void volume is created between the wearer's body and the bodyside liner and absorbent core of the absorbent article to help contain body exudates.

"Continuous" means that the described structure is a closed-loop structure. The continuous structure may be unitary, i.e., a one-piece structure, or may be made up of individual elements suitably joined together to form a closed-loop.

A "continuous waistband" can be an elastomeric, cloth-like, nonwoven fibrous material, such as an elastomeric stretch bonded laminate web or an elastomeric meltblown web. By proper selection of materials, the continuous waistband can be rendered temporarily elastically inhibited, such as by compression. Once temporarily elastically inhibited, the elastic material, of which waistband is comprised, can be activated, such as by treating with heat, to recover to a state of elasticity.

"Conventional hot-melt adhesive" means a formulation that generally comprises several components. These components typically include one or more polymers to provide cohesive strength (e.g., aliphatic polyolefins such as poly (ethylene-co-propylene) copolymer; ethylene vinyl acetate copolymers; styrene-butadiene or styrene- isoprene block copolymers; etc.); a resin or analogous material (sometimes called a tackifier) to provide adhesive strength (e.g., hydrocarbons distilled from petroleum distillates; rosins and/or rosin esters; terpenes derived, for example, from wood or citrus, etc.); perhaps waxes, plasticizers or other materials to modify viscosity (i.e., flowability) (examples of such materials include, but are not limited to, mineral oil, polybutene, paraffin oils, ester oils, and the like); and/or other additives including, but not limited to, antioxidants or other stabilizers. A typical hot-melt adhesive formulation might contain from about 15 to about 35 weight percent cohesive strength polymer or polymers; from about 50 to about 65 weight percent resin or other tackifier or tackifiers; from more than zero to about 30 weight percent plasticizer or other viscosity modifier; and optionally less than about 1 weight percent stabilizer or other additive. It should be understood that other adhesive formulations comprising different weight percentages of these components are possible.

The term "density" or "concentration" when referring to the absorbent material, in particular the SAP, of a layer, refers to the amount of the absorbent material divided by the surface area of the layer over which the absorbent material is spread out.

As used herein, the term "diaper" refers to an absorbent article generally worn by infants about the lower torso.

The term "disposable" is used herein to describe absorbent articles that generally are not intended to be laundered or otherwise restored or reused as an absorbent article (i.e., they are intended to be discarded after a single use and, preferably, to be recycled, composted or otherwise disposed of in an environmentally compatible manner).

As used herein, the term "elastic resistance" describes an elastic force that tends to resist an applied tensile force causing a material provided therewith to tend to contract to an untensioned configuration in response to a stretching force.

As used herein, the terms "elastic", "elastomeric", "elasticity" or derivations thereof are used to describe the ability of various materials and objects comprised of such to reversibly undergo deformation under stress, e.g., become stretched or extended, in at least one direction when a force is applied to the material and to resume substantially to their original dimensions upon relaxing, i.e., when the force is released, without rupture or breakage. Preferably, it refers to a material or composite which can be elongated in at least one direction by at least 50% of its relaxed length, i.e., elongated to at least 150% of its relaxed length, and which will recover upon release of the applied tension at least 40% of its elongation. Accordingly, upon release of the applied tension at 50% elongation, the material or composite contracts to a relaxed length of not more than 130% of its original length. Examples of suitable elastomer materials include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

The term "elasticized" refers to a material, layer, or substrate that is naturally non-elastic, but which has been rendered elastic by, for example, suitably joining an elastic material, layer, or substrate thereto.

"Elongation" means the ratio of the extension of a material to the length of the material prior to the extension (expressed in percent), as represented by the following: "Extension" means the change in length of a material due to stretching (expressed in units of length).

As used herein the term "extensible" means elongatable in at least one direction, but not necessarily recoverable.

The term "fabrics" is used to refer to all of the woven, knitted and nonwoven fibrous webs.
"Fastening means", such as tape tab fasteners, are typically applied to the back waistband region of the diaper to provide a mechanism for holding the diaper on the wearer. Fastening means, such as tape tab fasteners, snaps, pins, belts, hooks, buckles, "hook/mushroom"-and-loop fasteners (e.g. VELCRO®-type fasteners) and the like, may be employed and are typically applied at the lateral, side ends of the back waistband region of diaper to provide a mechanism for holding the diaper about the waist of the wearer in a conventional manner. Tape tab fasteners can be any of those well known in the art, and are typically applied to the corners of the diaper. For example, adhesive fasteners, mechanical fasteners, hook-and-loop fasteners, snaps, pins or buckles, may be used alone, or in combination. For example, the fasteners can be adhesive fasteners, which are constructed to releasably adhere to a landing zone patch attached to the front waistband section of the diaper to provide a refastenable adhesive fastening system.

The term "finished" or "final", when used with reference to a product, means that the product has been suitably manufactured for its intended purpose.

The term "flexible" refers to materials which are compliant and which will readily conform to the general shape and contours of the wearer's body.

As used herein, the term "garment" means any type of apparel which may be worn. This includes diapers, training pants, incontinence products, surgical gowns, industrial workwear and coveralls, undergarments, pants, shirts, jackets and the like.

Many of the known superabsorbent polymer particles exhibit gel blocking. "Gel blocking" occurs when superabsorbent polymer particles are wetted and the particles swell so as to inhibit fluid transmission to other regions of the absorbent structure. Wetting of these other regions of the absorbent member therefore takes place via a very slow diffusion process. In practical terms, this means acquisition of fluids by the absorbent structure is much slower than the rate at which fluids are discharged, especially in gush situations. Leakage from the absorbent article can take place well before the particles of SAP in the absorbent member are even close to being fully saturated or before the fluid can diffuse or wick past the "blocking" particles into the rest of the absorbent member. Gel blocking can be a particularly acute problem if the superabsorbent polymer particles do not have adequate gel strength and deform or spread under stress once the particles swell with absorbed fluid.

The term "graphic" includes, but is not limited to, any type of design, image, mark, figure, codes, words, patterns, or the like. For a product such as a training pant, graphics will generally include objects associated with little boys and little girls, such as multi-color trucks, airplanes, balls, dolls, bows, or the like.

"Hydroentanglement process" refers to the manufacturing of nonwoven webs. The process involves directing a series of water jets towards a fibrous web which is supported on a moving porous belt. The water jets pass downwards through the mass of fibres and on making contact with the surface of the belt, the jets rebound, and break up: the energy released causes entanglement of the mass of fibres.

The term "high-absorbency material" refers to materials that are capable of absorbing at least 10 times their own weight in liquid. The high-absorbency material may comprise absorbent gelling materials, such as superabsorbent polymers. Superabsorbent polymers are water-swellable, water-insoluble organic or inorganic materials capable of absorbing at least about 20 times their own weight of an aqueous solution containing 0.9 weight percent of sodium chloride. Absorbent gelling materials can be natural, synthetic and modified natural polymers and materials. In addition, the absorbent gelling materials can be inorganic materials, such as silica gels, or organic compounds such as cross-linked polymers. The term "cross-linked" refers to any means for effectively rendering normally water-soluble materials substantially water insoluble but swellable. Such means can include, for example, physical entanglement, crystalline domains, covalent bonds, ionic complexes and associations, hydrophilic associations, such as hydrogen bonding, and hydrophobic associations or Van der Waals forces. Examples of synthetic absorbent gelling material polymers include the alkali metal and ammonium salts of poly(acrylic acid) and poly (methacrylic acid), poly(acrylamides), poly(vinyl ethers), maleic anhydride copolymers with vinyl ethers and alpha-olefins, poly(vinyl pyrrolidone), poly(vinylmorpholinone), poly(vinyl alcohol), and mixtures and copolymers thereof. Further polymers suitable for use in the absorbent structure include natural and modified natural polymers, such as hydrolyzed acrylonitrile-grafted starch, acrylic acid grafted starch, methyl cellulose, carboxymethyl cellulose, hydroxypropyl cellulose, and the natural gums, such as alginates, xanthan gum, locust bean gum and the like. Mixtures of natural and wholly or partially synthetic absorbent polymers can also be used. Synthetic absorbent gelling materials typically are xerogels which form hydrogels when wetted. The term "hydrogel", however, has commonly been used to also refer to both the wetted and unwetted forms of the material. The high-absorbency material may be in any of a wide variety of geometric forms. As a general rule, it is preferred that the high-absorbency material be in the form of discrete particles. However, the high-absorbency material may also be in the form of fibres, flakes, rods, spheres, needles, spiral or semi-spiral, cubic, rod-like, polyhedral, or the like. Conglomerates of particles of high-absorbency material may also be used. The high-absorbency material may be present in the absorbent core in an amount of from about 5 to about 100 weight percent and desirably from about 30 to about 100 weight percent based on the total weight of the absorbent core. The distribution of the high-absorbency material within the different portions of the absorbent core can vary depending upon the intended end use of the absorbent core. The high-absorbency material may be arranged in a generally discrete layer within the matrix of hydrophilic fibres. Alternatively, the absorbent core may comprise a laminate of fibrous webs and high-absorbency material or other suitable means of maintaining a high-absorbency material in a localized area.

A "hook-and-loop fastener" refers to complementary fastening means having a "hook" portion and a "loop" portion and which are refastenable. The term "hook" as used herein refers to any element capable of engaging another element, the so called "loop" portion. The term "hook" is not limited to only "hooks" in its normal sense, but rather encompasses any form of engaging elements, whether unidirectional or bi-directional. The term "loop" is likewise not limited to "loops" in its normal sense, but also encompasses any structure capable of engaging with a "hook" fastener. Examples of "loop" materials are fibrous structures, like nonwoven materials.

The term "hydrophilic" describes fibers or the surfaces of fibers which are wetted by the aqueous liquids in contact with the fibers. The degree of wetting of the materials can, in turn, be described in terms of the contact angles and the surface tensions of the liquids and materials involved. The term "wettable" is meant to refer to a fiber which exhibits a liquid, such as water, synthetic urine, or a 0.9 weight percent aqueous saline solution, in air contact angle of less than 90°, whereas "hydrophobic" or "non-wettable" describes fibers having contact angles equal to or greater than 90°.

As used herein, the term "impermeable" generally refers to articles and/or elements that are substantially not penetrated by aqueous fluid through the entire thickness thereof under a pressure of 1.0 kPa or less. Preferably, the impermeable article or element is not penetrated by aqueous fluid under pressures of 3.4 kPa or less. More preferably, the impermeable article or element is not penetrated by fluid under pressures of 6.8 kPa or less. An article or element that is not impermeable is permeable.

"Integral" is used to refer to various portions of a single unitary element rather than separate structures bonded to or placed with or placed near one another.

"Join", "joining", "joined", or variations thereof, when used in describing the relationship between two or more elements, means that the elements can be connected together in any suitable manner, such as by heat sealing, ultrasonic bonding, thermal bonding, by adhesives, stitching, or the like. Further, the elements can be joined directly together, or may have one or more elements interposed between them, all of which are connected together.

The term "laid-flat state" is intended to refer to the article when it is flattened into a plane or is substantially flattened into a plane and is used in contrast to when the article otherwise positioned, such as when the article is folded or shaped in or for use by a wearer.

"Laminate" refers to elements being attached together in a layered arrangement.

The use of the term "layer" can refer, but is not limited, to any type of substrate, such as a woven web, nonwoven web, films, laminates, composites, elastomeric materials, or the like. A layer can be liquid and air permeable, permeable to air but impermeable to liquids, impermeable both to air and liquid, or the like. When used in the singular, it can have the dual meaning of a single element or a plurality of elements.

The crotch portion of the absorbent article preferably comprises opposite longitudinal side portions which comprise a pair of elasticized, longitudinally-extending "leg cuffs". The leg cuffs are generally adapted to fit about the legs of a wearer when in use and serve as a mechanical barrier to the lateral flow of body exudates. Leg cuffs are elasticized by leg elastics. The diaper further can comprise a front waist elastic and a rear waist elastic. Materials suitable for use in forming leg elastics are known to those skilled in the art. Exemplary of such materials are strands or ribbons of a polymeric, elastomeric material which are adhered to the diaper at the leg cuff while in a stretched position, or which are attached to the diaper while the diaper is pleated, such that elastic constrictive forces are imparted to the leg cuff. Examples of suitable elastomer materials that can be used include polyether-polyamide block copolymers, polyurethanes, synthetic linear A-B-A and A-B block copolymers, chlorinated rubber/EVA (ethylene-vinyl acetate) blends, EPDM (ethylene-propylene diene monomer) rubbers, EPM (ethylene-propylene monomer) rubbers, blends of EPDM/EPM/EVA, and the like.

"Liquid" means a nongaseous substance and/or material that flows and can assume the interior shape of a container into which it is poured or placed.

"Longitudinal" is a direction running parallel to the maximum linear dimension of the article.

The term "meltblown fibers" means fibers formed by extruding a molten thermoplastic material through a plurality of fine, usually circular, die capillaries as molten threads or filaments into a high velocity gas stream (e.g. air) which attenuates the filaments of molten thermoplastic material to reduce their diameter, which may be to microfiber diameter. In general, meltblown fibers have an average fiber diameter of up to about 10 microns. After the fibers are formed, the meltblown fibers are carried by the high velocity gas stream and are deposited on a collecting surface to form a web of randomly disbursed meltblown fibers.

The term "nonelastic" refers to any material which does not fall within the definition of "elastic" above
The term "nonwoven fabric or web" means a sheet material having a structure of individual fibers or threads which are interlaid, but not in a regular manner such as occurs with knitting or weaving processes. Nonwoven fabrics or webs have been formed from many processes such as for example, meltblowing processes, spunbonding processes, and bonded carded web processes.

By the terms "particle", "particles", "particulate", "particulates" and the like, it is meant that the material is generally in the form of discrete units. The units can comprise granules, powders, spheres, pulverized materials or the like, as well as combinations thereof. The particles can have any desired shape such as, for example, cubic, rod-like, polyhedral, spherical or semi-spherical, rounded or semi-rounded, angular, irregular, etc. Shapes having a large greatest dimension/smallest dimension ratio, like needles, flakes and fibers, are also contemplated for inclusion herein. The terms "particle" or "particulate" may also include an agglomeration comprising more than one individual particle, particulate or the like. Additionally, a particle, particulate or any desired agglomeration thereof may be composed of more than one type of material.

The term "polymer" generally includes, but is not limited to, homopolymers, copolymers, such as, for example, block, graft, random and alternating copolymers, terpolymers, etc. and blends and modifications thereof. Furthermore, unless otherwise specifically limited, the term "polymer" shall include all possible geometrical configurations of the material. These configurations include, but are not limited to, isotactic, syndiotactic and random symmetries.

By the term "pre-packed" as used herein, is meant that one or more absorbent articles are packed in a single unit before being stacked.

"Pulp fluff" or "fluff pulp" refers to a material made up of cellulose fibers. The fibers can be either natural or synthetic, or a combination thereof. The material is typically lightweight and has absorbent properties.

"Refastenable" refers to the property of two elements being capable of releasable attachment, separation, and subsequent releasable reattachment without substantial permanent deformation or rupture.

The "retention portion" or "liquid absorption layer" is part of the absorbent medium. This portion may comprise a matrix of hydrophilic fibers, such as a web of cellulosic fluff, mixed with particles of high-absorbency material. In particular arrangements, the retention portion may comprise a mixture of superabsorbent hydrogel-forming particles and synthetic polymer meltblown fibers, or a mixture of superabsorbent particles with a fibrous coform material comprising a blend of natural fibers and/or synthetic polymer fibers. The superabsorbent particles may be substantially homogeneously mixed with the hydrophilic fibers, or may be nonuniformly mixed. For example, the concentrations of superabsorbent particles may be arranged in a non-step-wise gradient through a substantial portion of the thickness of the absorbent structure, with lower concentrations toward the bodyside of the absorbent structure and relatively higher concentrations toward the outerside of the absorbent structure. The superabsorbent particles may also be arranged in a generally discrete layer within the matrix of hydrophilic fibers. In addition, two or more different types of superabsorbent may be selectively positioned at different locations within or along the fiber matrix.

As used herein the term "sheet" or "sheet material" refers to woven materials, nonwoven webs, polymeric films, polymeric scrim-like materials, and polymeric foam sheeting.

The absorbent article may also contain side panels. The "side panels" can have any shape such as but not limited to square, rectangular, triangular, circular and trapezoidal shape. They can be joined to the respective opposite side portions of the back section, by a known method, such as heat-sealing or adhesive bonding. The side panels may also be formed integrally with the back section by projecting and joining together the respective top sheet and/or back sheet and/or absorbent medium outward in lugs having the shape of the side panels. Preferably, the side panels are formed by laminating a layer of nonwoven fabric, a layer of thermoplastic film and a layer of elastic material. The layer of elastic material might be sandwiched between the nonwoven fabric layer and the thermoplastic film by adhesive layers. The layer of nonwoven fabric might be made of natural fibers, synthetic fibers or a blend of natural fibers and synthetic fibers. The layer of thermoplastic film might be made of polyethylene or polypropylene.

The term "spunbond fibers" refers to fibers formed by extruding molten thermoplastic polymers as filaments or fibers from a plurality of relatively fine, usually circular, capillaries of a spinneret, and then rapidly drawing the extruded filaments by an eductive or other well-known drawing mechanism to impart molecular orientation and physical strength to the filaments. The average diameter of spunbond fibers is typically in the range of from 15-60 µm or higher. The spinneret can either be a large spinneret having several thousand holes per meter of width or be banks of smaller spinnerets, for example, containing as few as 40 holes.

The term "spunbond meltblown spunbond" (SMS) nonwoven fabric as used herein refers to a multi-layer composite sheet comprising a web of meltblown fibers sandwiched between and bonded to two spunbond layers. A SMS nonwoven fabric can be formed in-line by sequentially depositing a first layer of spunbond fibers, a layer of meltblown fibers, and a second layer of spunbond fibers on a moving porous collecting surface. The assembled layers can be bonded by passing them through a nip formed between two rolls that can be heated or unheated and smooth or patterned. Alternately, the individual spunbond and meltblown layers can be pre-formed and optionally bonded and collected individually such as by winding the fabrics on wind-up rolls. The individual layers can be assembled by layering at a later time and bonded together to form a SMS nonwoven fabric. Additional spunbond and/or meltblown layers can be incorporated in the SMS fabric, for example spunbond-meltblown-meltblown-spunbond (SMMS), etc.

"Staple fibers" refer to commercially available fibers having diameters ranging from less than about 0.001 mm to more than about 0.2 mm; they come in several different forms such as short fibers ranging from about 10 to 50 mm in length and long fibers with a length higher than 50 mm, preferably up to 100 mm.

By "stretch", it is meant that the material has the ability to extend beyond its original size in at least one dimension when subjected to a tensile force (i.e., tension) applied in the direction of that dimension, without breaking the material. An extension of for example 50% means that the material with an initial length of 100mm has reached a length of 150mm. Stretch may be unidirectional, bi-directional, or multi-directional. The specific stretch properties of a material may vary along any of the stretch vectors. The term can include elastic materials, as well as nonwovens that can be inherently extensible, but not necessarily in an elastic manner. Such nonwovens can be made to behave in an elastic manner by bonding them to elastic films.
Use of the term "substrate" includes, but is not limited to, woven or nonwoven webs, porous films, ink permeable films, paper, composite structures, or the like.

Superabsorbent materials suitable for use in the present invention are known to those skilled in the art, and may be in any operative form, such as particulate form, fibers and mixtures thereof. Generally stated, the "superabsorbent material" can be a water-swellable, generally water-insoluble, hydrogel-forming polymeric absorbent material, which is capable of absorbing at least about 15, suitably about 30, and possibly about 60 times or more its weight in physiological saline (e.g. saline with 0.9 wt % NaCI). The superabsorbent material may be biodegradable or bipolar. The hydrogel-forming polymeric absorbent material may be formed from organic hydrogel-forming polymeric material, which may include natural material such as agar, pectin, and guar gum; modified natural materials such as carboxymethyl cellulose, carboxyethyl cellulose, and hydroxypropyl cellulose; and synthetic hydrogel-forming polymers. Synthetic hydrogel-forming polymers include, for example, alkali metal salts of polyacrylic acid, polyacrylamides, polyvinyl alcohol, ethylene maleic anhydride copolymers, polyvinyl ethers, polyvinyl morpholinone, polymers and copolymers of vinyl sulfonic acid, polyacrylates, polyacrylamides, polyvinyl pyridine, and the like. Other suitable hydrogel-forming polymers include hydrolyzed acrylonitrile grafted starch, acrylic acid grafted starch, and isobutylene maleic anhydride copolymers and mixtures thereof. The hydrogel-forming polymers may be lightly crosslinked to render the material substantially water insoluble. Crosslinking may, for example, be by irradiation or covalent, ionic, Van der Waals, or hydrogen bonding. The superabsorbent material may suitably be included in an appointed storage or retention portion of the absorbent system, and may optionally be employed in other components or portions of the absorbent article. The superabsorbent material may be included in the absorbent layer or other fluid storage layer of the absorbent article of the present invention in an amount up to about 60% by weight. Typically, the superabsorbent material, when present, will be included in an amount of about 5% to about 40% by weight, based on the total weight of the absorbent layer.

"Superabsorbent polymer particles" or "SAPs" refer to water-swellable, water-insoluble organic or inorganic materials capable, under the most favorable conditions, of absorbing at least about 10 times their weight, or at least about 15 times their weight, or at least about 25 times their weight in an aqueous solution containing 0.9 weight percent sodium chloride. In absorbent articles, such as diapers, incontinent diapers, etc., the particle size is typically ranging between 100 to 800 µm, preferably between 300 to 600 µm, more preferably between 400 to 500 µm.

The term "target zone" refers to an area of an absorbent core where it is particularly desirable for the majority of a fluid insult, such as urine, menses, or bowel movement, to initially contact. In particular, for an absorbent core with one or more fluid insult points in use, the insult target zone refers to the area of the absorbent core extending a distance equal to 15% of the total length of the composite from each insult point in both directions.

"Tension" includes a uniaxial force tending to cause the extension of a body or the balancing force within that body resisting the extension.

As used herein, the term "thermoplastic" is meant to describe a material that softens when exposed to heat and which substantially returns to its original condition when cooled to room temperature.

The term "top sheet" refers to a liquid permeable material sheet forming the inner cover of the absorbent article and which in use is placed in direct contact with the skin of the wearer. The top sheet is typically employed to help isolate the wearer's skin from liquids held in the absorbent structure. The top sheet can comprise a nonwoven material, e.g. spunbond, meltblown, carded, hydroentangled, wetlaid etc. Suitable nonwoven materials can be composed of man-made fibres, such as polyester, polyethylene, polypropylene, viscose, rayon etc. or natural fibers, such as wood pulp or cotton fibres, or from a mixture of natural and man-made fibres. The top sheet material may further be composed of two fibres, which may be bonded to each other in a bonding pattern. Further examples of top sheet materials are porous foams, apertured plastic films, laminates of nonwoven materials and apertured plastic films etc. The materials suited as top sheet materials should be soft and non-irritating to the skin and be readily penetrated by body fluid, e.g. urine or menstrual fluid. The inner coversheet may further be different in different parts of the absorbent article. The top sheet fabrics may be composed of a substantially hydrophobic material, and the hydrophobic material may optionally be treated with a surfactant or otherwise processed to impart a desired level of wettability and hydrophilicity.

As used herein, the term "transverse" or "lateral" refers to a line, axis, or direction which lies within the plane of the absorbent article and is generally perpendicular to the longitudinal direction.

"Ultrasonic welding" refers to a technology which joins two materials by melting them with heat generated from ultrasonic oscillation and then laminating them together, such that the molten materials flow and fill the gap between the two unaffected portions of the two materials, respectively. Upon cooling and shaping, the two materials are joined together.

As used herein, the term "water-swellable, water-insoluble" is meant to refer to a material that, when exposed to an excess of water, swells to its equilibrium volume but does not dissolve into the solution. As such, a water-swellable, water-insoluble material generally retains its original identity or physical structure, but in a highly expanded state, during the absorption of the water and, thus, must have sufficient physical integrity to resist flow and fusion with neighboring particles.

By the term "wrapping material" as used herein, is meant a bendable material, preferably a sheet material of which the thickness is smaller, more preferably much smaller than its width or length, such as a sheet, a film or a foil. In a particularly preferred embodiment, said wrapping material is capable of being rolled up.

Due to the high concentrations of superabsorbent particles, or other high-absorbency material, in the retention portion, there can be an increased difficulty with regard to containing the high-absorbency particles within the retention portion and restricting the movement or migration of the superabsorbent onto the bodyside of the diaper. To improve the containment of the high-absorbency material, the absorbent structure can include an improved overwrap, such as a "wrap sheet", placed immediately adjacent and around the retention portion. The wrap sheet is preferably a layer of absorbent material which covers the major bodyside and outerside surfaces of the retention portion, and preferably encloses substantially all of the peripheral edges of the retention portion to form a substantially complete envelope thereabout. Alternatively, the wrap sheet can provide an absorbent wrap which covers the major bodyside and outerside surfaces of the retention portion, and encloses substantially only the lateral side edges of the retention portion. Accordingly, both the linear and the inwardly curved portions of the lateral side edges of the wrap sheet would be closed about the retention portion. In such an arrangement, however, the end edges of the wrap sheet may not be completely closed around the end edges of the retention portion at the waistband regions of the article. The wrap sheet may comprise a multi-element wrapsheet which includes a separate bodyside wrap layer and a separate outerside wrap layer, each of which extends past all or some of the peripheral edges of the retention portion. Such a configuration of the wrap sheet can, for example, facilitate the formation of a substantially complete sealing and closure around the peripheral edges of the retention portion. The bodyside and outerside layers of the wrap sheet may be composed of substantially the same material, or may be composed of different materials. For example, the outerside layer of the wrap sheet may be composed of a relatively lower basis weight material having a relatively high porosity, such as a wet strength cellulosic tissue composed of softwood pulp. The bodyside layer of the wrap sheet may comprise one of the previously described wrap sheet materials (for example may comprise a meltblown web composed of meltblown polypropylene fibers or low porosity cellulosic tissue web composed of a blend of hardwood/softwood fibers) which has a relatively low porosity. The low porosity bodyside layer can better prevent the migration of superabsorbent particles onto the wearer's skin, and the high porosity, lower basis weight outerside layer can help reduce costs.

Illustrative embodiments of the present invention are further explained by way of example only, and not for limitation, with respect to the accompanying drawings in which like reference numerals refer to similar elements. Similar entities and reference numbers in different figures have been partially omitted. It is also mentioned that the various features are not necessarily drawn to scale.

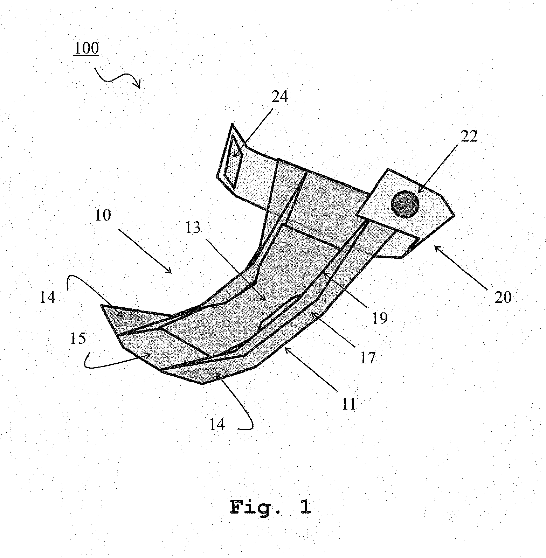

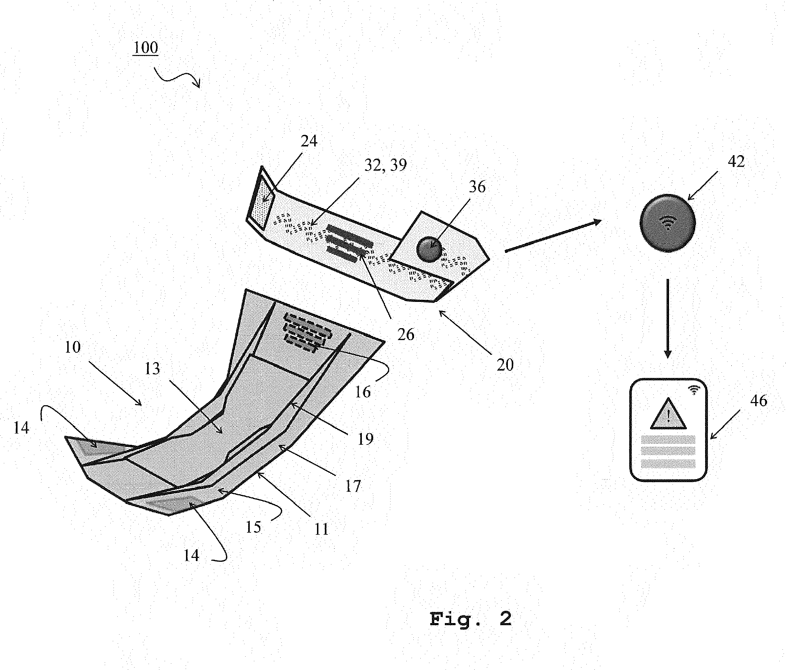

In a preferred embodiment, depicted in Figs. 1 and 2, the present invention provides an absorbent article 100 comprising a first part being a disposable part 10, and a second part being a re-usable part 20, wherein the re-usable part 20 is configured to removably hold the disposable part 10, and wherein the absorbent article 100 comprises an electronic system 22 configured to measure data related to the absorbent article 100 and/or to ambient conditions in close proximity to a wearer.

Combining the re-usable part 20 and the disposable part 10 is required to form a fully functional absorbent article. The absorbent article 100 as a whole is meant to provide protection against leakage of body fluids for the wearer, for example a baby or an adult. The functional components required for that purpose, as known by the skilled artisan, include a chassis 11 and an absorbent core 13. Moreover, the chassis 11 includes a back sheet 15, a top sheet 17, and preferably barrier leg cuffs 19 and leg elastics.

The present invention encompasses different ways in which some of these functional components may be included in the disposable part 10 and others may be included in the re-usable part 20, in a manner that the disposable part 10 can be easily and securely attached and detached from the re-usable part 20.

In an embodiment, the disposable part 10 comprises at least the absorbent core 13, whereas the re-usable part 20 is configured to extend along at least a width of the disposable part 10 and to extend along at least a portion of a length of the disposable part 10. In this fashion, when the absorbent article is put on a wearer, the re-usable part 20 extends around the entire waist of the disposable part 10 of the absorbent article and securely supports said disposable part 10. In addition to this, the disposable part 10 comprises at least the absorbent core 13.

In a preferred embodiment, shown in Figs. 1 and 2, the re-usable part 20 comprises an elongated part in the form of a belt, whereas the disposable part 10 comprises the chassis 11 (and thereby the back sheet 15, the top sheet 17, the barrier leg cuffs 19 and leg elastics) and the absorbent core 13. Preferably, the re-usable part 20 is washable and/or is made of textile material. In addition to this or as an alternative, at least portions of said re-usable part 20 have elastic properties aimed to improve fitting to the wearer.

Furthermore, in this preferable embodiment, the re-usable part 20 may comprise some elements 24 of a fastening system of the absorbent article 100 whilst the disposable part 10 comprises complementary elements 14 of the fastening system, as depicted in Figs. 1 and 2. Said elements of the fastening system may be, by way of example, hook fasteners or loop elements. The hook fasteners may be comprised in the re-usable part 20 and corresponding loop elements may be comprised in the disposable part 10. Alternatively, hook fasteners may be comprised in the disposable part 10 while corresponding loop fasteners may be comprised in the re-usable part 10, or any combination thereof.

By way of further example, micro hook and loop fasteners can be used. Alternative materials commonly used for fastening absorbent articles may be used and combined to securely fastening the absorbent article 100.

In this manner, as mentioned before, combining both re-usable 20 and disposable 10 parts are required for properly closing and fitting the absorbent article when worn by a user, e.g. by a baby or by an adult. Thus, the re-usable part 20 in general may comprise elements of the fastening system that are not included in the disposable part 10 to ensure proper fastening. In this context, it is also mentioned that the fastening elements may be implemented as a continuous component placed along the width of the re-usable part 20 whereas complementary elements may be placed also continuously along the width of the disposable part 10. Non-continuous elements may also be used to that end.

Alternatively, the re-usable part 20 may comprise the fastening system of the absorbent article. As a further alternative, the disposable part 10 may comprise the fastening system for the absorbent article 100. In this way, the re-usable part 20 is also configured to be used in combination with other disposable articles, as baby diapers or adult diapers, that can be commonly acquired in the market.

In the context of the preferred embodiment, the absorbent article 100 further comprises at least one holding means 16, 26 configured to attach the re-usable part 20 to the disposable part 10, as depicted in Fig. 2. It should also be mentioned that said holding means 16 can be present entirely on the disposable part 10. Alternatively, the holding means 26 can be present entirely on the re-usable part 20. As a further, preferable alternative, the holding means 16, 26 may be present on both disposable 10 and re-usable 20 parts, respectively.

By way of example, the holding means 16, 26 may be implemented in the form of a hook and loop system, whereby the hook elements may be comprised in the re-usable part 20 whereas the loop elements are comprised in the disposable part 10, or vice versa. Moreover, the hook (or loop) elements 26 may be embodied as a continuous portion on a side of the re-usable part 20 facing the disposable part 10 and the body of a wearer, whilst the corresponding loop (or hook) element 16 may be located on an outer side of the back sheet 15 of the chassis 11 and arranged proximal to a garment facing side of said absorbent article 100. Alternatively, the loop (or hook) element 16 may consist of two parts, each of which is placed at opposite front and back ends of the chassis 11 in a manner that, when the absorbent article 100 is worn, both re-usable 20 and disposable 10 parts are securely attached to each other along the entire waist of the absorbent article and of the wearer. By way of further example, the holding means 16 comprised in the re-usable part may consist of only a single portion in either the back end or the front end of the chassis 11. Yet as a further example, the hook and loop elements of the holding means 16, 26 may be formed as a plurality of sections placed along the width of the re-usable part 20 and of the disposable part 10.

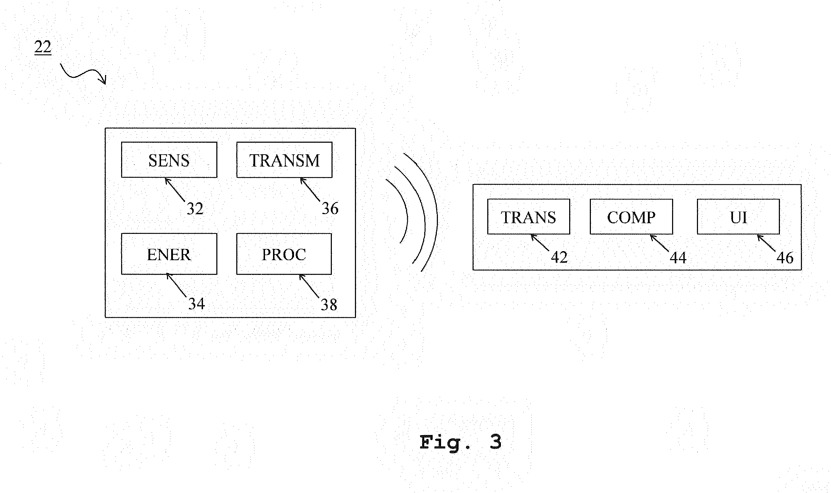

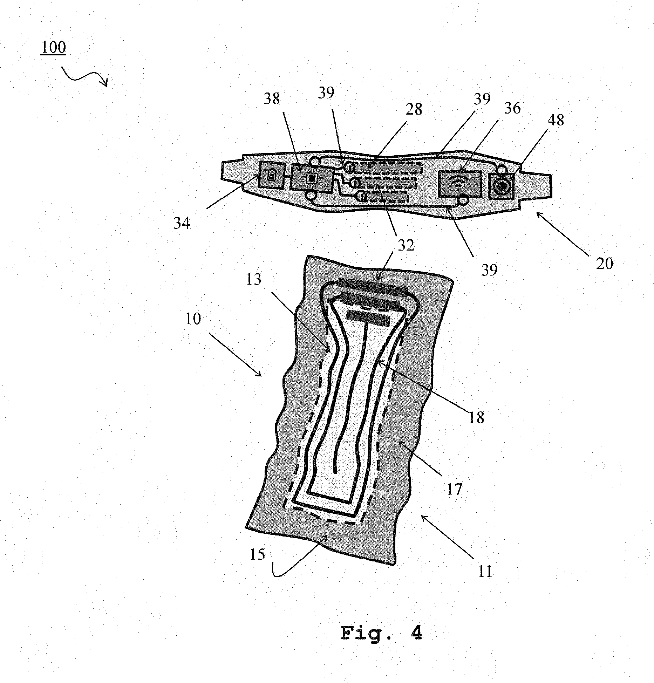

As mentioned above, the absorbent article 100 comprises the electronic system 22. A preferred embodiment for said electronic system 22 is depicted in Fig. 3, in which it can be noted that the electronic system 22 comprises at least one type of sensor 32 and a transmitter 36, for transmitting measured data related to the absorbent article. Additionally or alternatively, for transmitting measured data related to ambient conditions in close proximity to a wearer. The measured data may be achieved either by conductive or non-conductive measurements performed by the at least one type of sensor 32. Preferably, the electronic system 22 also comprises a data processing unit 38. It may be further advantageous that the electronic system 22 also comprises an energy harvester 34. Said energy harvester may be, for example, an electric power source. Integrated electronics 39 (shown in Fig. 4) connecting different parts of the electronic system 22 may also be envisaged.

In a preferred embodiment, the at least one sensor 32 is a humidity sensor or a wetness sensor, configured to detect presence of wetness due to liquids and/or exudates. The electronic system 22 may include additional sensors for measuring ambient parameters and/or body parameters, or any combination thereof. The mentioned parameters may comprise at least one of humidity, wetness, gas concentration, acceleration, body orientation (detected by means of a gyroscope), body temperature, heart rate, blood pressure, ambient temperature, air pressure, electric field strength, magnetic field strength, volume of sound, radiation dose, or any combination thereof.

In an embodiment, the electronic system 22 or at least one of its elements is included in a device separated from the re-usable part 20 and the disposable part 10, in a manner that it is mechanically connected to the absorbent article. An example for such a device is a clip-on module device, as known from the prior art, that can be inserted into a pocket which is integrated either into the re-usable 20 part or into the disposable part 10, or by using hook and loop attachment between the re-usable 20 and disposable 10 parts, or by a combination thereof.

Additionally or alternatively, the electronic system 22, or at least one element of the electronic system 22, can be integrated into the re-usable part 20 by making use of the technology known as "smart textiles", "smart garments", "flexible electronics" or "wearable electronics" 39. In addition to this or as a further alternative, the electronic system 22, or at least one element of the electronic system may be integrated into the disposable part 10, for example in the form of conductive circuits printed onto a substrate of the chassis 11 or onto nonwoven components of the chassis 11.

Combinations of the previous alternatives for incorporating at least some of the elements of the electronic system 22 in either the re-usable part 20 or the disposable part 10 can also be considered. By way of example, the holding means 16, 26 may also be used not only to achieve mechanical attachment between the re-usable 20 and disposable 10 parts, but also to achieve electrical interconnection between elements of the electronic system 22.

In an embodiment, shown in Fig. 4, an exemplary wetness sensor 32 may comprise a conductive pattern 18 having several connection tracks printed onto a surface of the back sheet 15 of the chassis 11. The connection tracks 18 can be brought in electrical communication with corresponding electrical terminals 28 which are configured to measure resistance, impedance and/or capacitance therethrough. The terminals 28 may be incorporated into the re-usable part 20 by using "flexible electronics". In this manner, the wetness sensor exemplified in the embodiment of Fig. 4 is comprised partially into the disposable part 10 and partially into the re-usable part 20 and, furthermore, such a sensor 32 also comprises holding means for attaching both re-usable 10 and disposable 20 parts to each other.

Moreover, Fig. 4 also shows that other elements of the electronic system 22, by way of example an energy harvester 34 in the form of an integrated battery, the integrated processing unit 36 and a transmitter 38, are integrated into the re-usable part 20 by using "flexible electronics" 39. From Fig. 4 it can be noted that the electronic system 22 may further comprise a control element 48, for example a press button or the like. It is also mentioned that the electronic system 22 comprises, in general, a conductive or non-conductive interface between the re-usable 20 and disposable 10 parts.

In the exemplary embodiment of Fig. 4, the electrical terminals 28 are located on a side of the re-usable part 20 facing the back end of the chassis 11 of the disposable part 10. Additionally or alternatively, electrical terminals may also be located in the side of the re-usable part 20 facing the front end of the chassis 11. In this manner, wetness detection is achieved by performing conductive measurements from front to back ends of the disposable article 100 when worn by a user.

Alternatively, instead of using the electrical terminals 28 within the re-usable part 20, the connection tracks 18 may be brought in electrical communication with a clip-on module device external to both re-usable 20 and disposable 10 parts, said clip-on module device being configured to measure resistance, impedance and/or capacitance therethrough. Nevertheless, integrating some elements of the electronic system 22 into a "smart textile" of the disposable part 20 is beneficial compared to the use of a removable clip-on module device, since said clip-on device may fit into a baby's mouth whereas the disposable part 20 does not fit and hence, advantageously, overcomes the safety risk for a wearer. In addition to this safety aspect, the comfort for the wearer is improved by including the electronics in the form of "smart textiles" instead of using a rigid clip-on device. Even with a full smart textile approach though, some rigid components, although small of size, might be required. Also, some removable components may be required in order to achieve washing survivability.

By way of further example, the absorbent core 13 of the disposable part 10 may be used as a wetness sensor 32 since, inherent to absorbent articles, a large portion of the core material becomes conductive once insults occur. Hence, conductive measurements of the absorbent core 13 may be used as an indicator of wetness. The conductive measurements can be achieved by incorporating electrical terminals, exemplary terminals 28 into the re-usable part 20 said terminals 28 being configured to measure resistance, impedance and/or capacitance through the core 13 without the need to include printed tracks 18. Moreover, in order to facilitate the terminals to detect wetness in the absorbent core 13, said core 13 may have a channel structure that enhances liquid distribution along the length of the disposable part 10. Additionally or alternatively, a wetness distributing element, for example a strip of hydrophilic nonwoven or the like element, can be incorporated into the absorbent core 13.

As an additional example, wetness detection may be achieved by optical means. In this context, the disposable part 10 may comprise a material, for example a type of ink or the like, which undergoes a change of color through its contact with body liquids and/or exudates via a chemical reaction. Such a material may be used as a wetness sensor 32, and the re-usable part 20 may comprise an optical detector capable to identify a change of color in the disposable part 10.

In yet a further example, the at least one sensor 32 may be incorporated into the fastening system of the absorbent article 100 by using conductive hook elements and/or conductive loop elements, said elements configured to perform wetness detection via conductive measurements. Additionally or as an alternative, said conductive fastening elements may be configured to detect changes in movement. For example the posture of the wearer can be determined. Moreover, bowel movements can be detected using such a sensor, and therefore it can be used to detect feces. This is beneficial especially for babies with sensitive skin.

As another example, a change of location of the wearer may be detected, and hence the sensor may be used as location sensor. For example, a location of a baby within a room or building may be determined. Also, a location of an elderly person outside may be detected.

In embodiments in which elements of the electronic system 22 are placed on distinct components of the absorbent article 100, it may be required that an electrical connection is properly established at the time of assembling the re-usable 20 and the disposable 10 parts to form the absorbent article 100. In that case, appropriate means, for example in the form of plugs or conductive elements, should be also incorporated.

By way of example, in order to ensure proper electrical connection of the connection tracks 18 of the disposable part 10 and the terminals 28 of the re-usable part 20, a clamping mechanism may be further employed. Said clamp may be designed in a manner that it does not only establish electrical contact but it also reinforces the mechanical attachment of the re-usable part 20 to the chassis 11 of the disposable part 10, thereby providing a stable and reliable fit of the absorbent article.

In an embodiment for the electronic system 22, depicted in Fig. 3, the at least one type of sensor 32 performs wetness and/or body parameters detection and thus sensor data is generated. The transmitter 36 may further provide a wireless connection to an external transceiver 42. The transceiver 42 may be connected to an external computing system 44 by which the sensor data are further processed. The electronic system 22 may also include a data processing unit 38, such as a microprocessor, which is in operative communication with the at least one sensor 32 and with the transmitter 36. The data processing unit 38 may use raw signals from the at least one type of sensor 32 to compute secondary data. Said secondary data are then sent to the external transceiver 42 by the transmitter 36. After the sensor data has been processed, the transceiver 42 is configured to send data to a user interface 41, for example in the form of an alert.

In addition, the electronic system 22 may include appropriate means for electrical connection between the different elements comprising the at least one type of sensor 32, the processing unit 38 and the transmitter 36. Those connection means may be in the form of direct electric contact, or it may be wireless, for example by transmitting and receiving electromagnetic waves or by transmitting radio frequency (RF) signals. Also an inductive transmission using coils is possible.

The utmost functionality of the electronic system 22 is to detect the presence of wetness and/or exudates in the absorbent article 100 and to provide an indication for a need for change of the absorbent article. Advantageously, skin dryness of the user is enhanced. Furthermore, optimizing the change of diapers has an advantageous economical and environmental impact by reducing the amount of diapers used and thereby the amount of waste created.

In an embodiment, the electronic system 22 may be also configured to provide connection with a user interface 46, for example a smartphone, via wireless connection or via radio frequency (RF) connection or via Bluetooth connection, or the like. In this manner, advantageously, the absorbent article herein disclosed may comprise further functionalities. The absorbent article 100 and its electronic system 22 may also be used to identify the type of product that is used, for example the size of the absorbent article or the level of absorbency or the like. Additionally or alternatively, it can be used to identify if the product is properly applied to the user by measuring, for example, the stretch level of certain elements of the fastening system or by indicating whether the leg cuffs are properly positioned. Additionally or alternatively, it can be used to provide a user or caregiver further instructions and/or background information. These examples may be achieved by means of further incorporating radio frequency identification (RFID) tags, QR codes or the like, in the re-usable 20 or disposable parts 10 or a combination thereof, to be detected by the electronic system 22.

Further advantageously, the functionality of the inventive absorbent article 100 is enhanced by incorporating additional types of sensors 32. For example, the absorbent article herein disclosed may be used for breathing monitoring (by using a stretch sensor that may be incorporated on the re-usable part 20 and/or on the disposable part 10) and/or for heart-rate monitoring and/or for body temperature measurement and monitoring and/or by detecting bowl movements or baby voice using a microphone as a sound sensor and/or for monitoring baby location by using a GPS tracker or a gyroscope and/or for activity monitoring or sleeping monitoring by using an acceleration sensor, or any combination thereof. In this manner, the invention enables a reliable monitoring of several health-related aspects of a particular user. Moreover, said health-related aspects may also be displayed in the user interface 46.

Each of the different type of sensors 32 may be placed either on the disposable part 10, or on the re-usable part 20, or a combination thereof, depending on the design and properties of each sensor 32. For example, a wetness sensor preferably covers a large portion of the surface of the disposable article 100 and, preferably, it is in contact with the liquid inside the absorbent core 13. As mentioned above, an example of this type of wetness sensors is a conductive pattern printed on the back sheet 15 of a chassis 11. Owing to the fact that conductive ink printing or other conductive elements are readily available at relatively low costs, such a wetness sensor may be preferably incorporated into the disposable part 10 of the absorbent article 100. In contrast, a gas sensor (aimed at detecting the presence of feces) is comparably more expensive and, moreover, typically comprises a semiconductor device. Thus, it is preferred to be re-usable and hence such a gas sensor may be configured to be incorporated into the re-usable part 20 of the absorbent article 100. In this manner, besides reducing costs by re-using such type of sensors, the environmental impact of the inventive absorbent article 100 is further decreased by avoiding to dispose semiconductor materials.

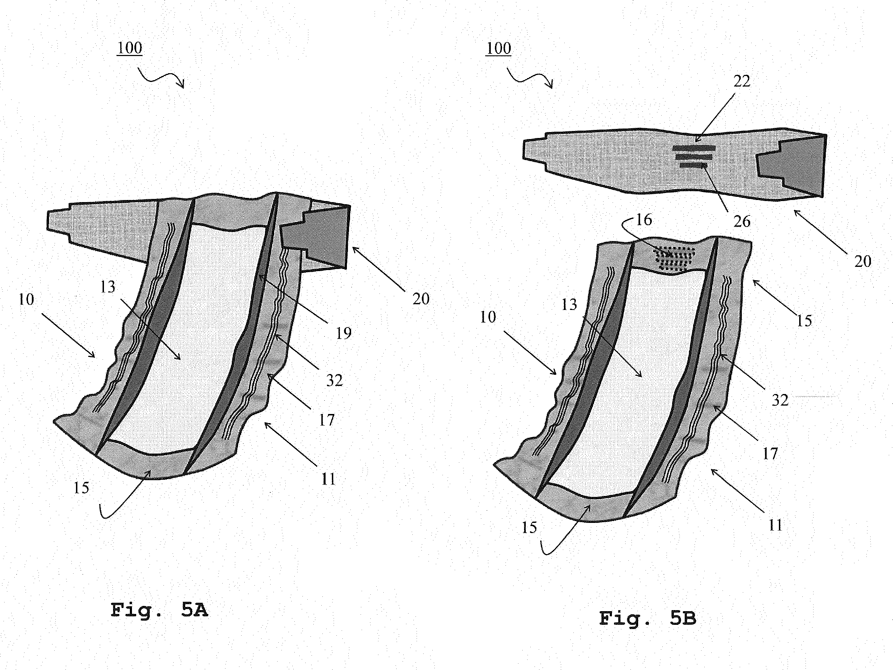

In a further exemplary embodiment, a sensor 32 may be implemented by using conductive elastic elements 32 into the chassis 11, as depicted in Figs. 5A and 5B. Said elastic conductive elements 32 may be incorporated into elastic strands. Additionally or alternatively, may be incorporated into the leg cuffs 19 to form elastic leg cuffs, or into an elastic waistband or the like known elastic components of an absorbent article. This type of sensors 32 are further brought in electrical contact to the rest of the elements of the electronic system 22, see Fig. 5B.

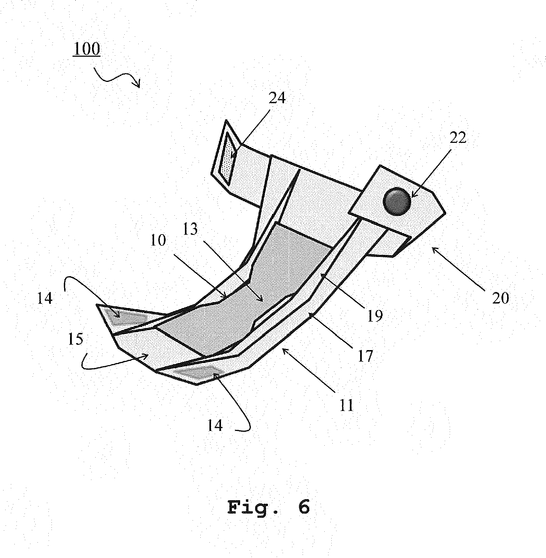

Figure 6 shows a further embodiment of the inventive absorbent article 100, in which the re-usable part 20 comprises at least a portion of the chassis 11 of the absorbent article 100 whereas the disposable part 10 comprises remaining portions of the chassis 11. Especially in the embodiment of Fig. 6, the re-usable part 20 comprises the entire back sheet 15, a part of the top sheet 17, the barrier leg cuffs 19, leg elastics and a fastening system 14, 24, whereas the disposable part includes the absorbent core 13 only. Further combinations of the components of the chassis 11 incorporated into the re-usable part 20 can also be comprised.

As discussed above, the absorbent article 100 comprises an electronic system 22, which in turn comprises at least one type of sensor 32 and a transmitter 36, for transmitting measured data related to the absorbent article and/or to ambient conditions in close proximity to a wearer. Also, the at least one type of sensor 32 is a humidity sensor and/or a wetness sensor and/or a sensor for measuring ambient parameters and/or a sensor for measuring body parameters and/or a sensor for measuring parameters of the absorbent article and/or any combination thereof.

Moreover, the electronic system 22 or at least one element of the electronic system 22 is i) included in a device separated from the re-usable part 20 and the disposable part 10, in a manner that it is mechanically connected to the absorbent article 100, and/or ii) is integrated into the re-usable part 20, and/or iii) is integrated into the disposable part 20, and/or any combination thereof.

The above-described examples and configurations for the fastening system 14, 24 as well as for the holding means 16, 26 configured to attach the disposable part 10 to the re-usable part 20, are also comprised in this embodiment.

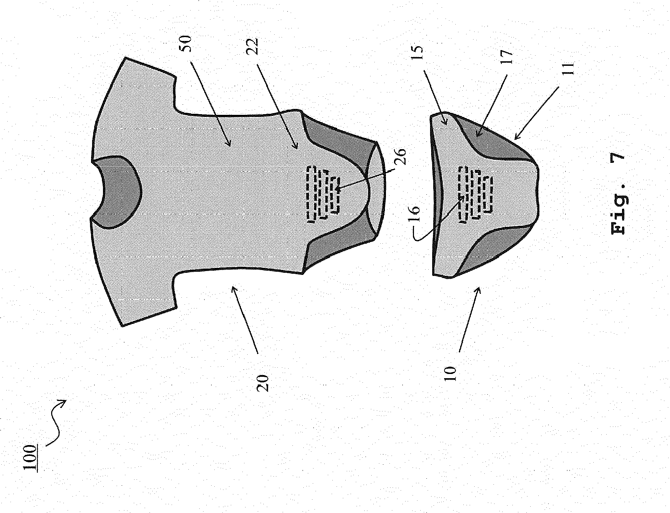

In yet a further embodiment of the inventive absorbent article 100, depicted in Fig. 7, the re-usable part 20 comprises a garment 50 to be worn by a user, especially a baby, whereas the disposable part 10 comprises the chassis 11 and the absorbent core 13. The garment 50 can be, for example, a baby body suit or a romper suit or the like. The absorbent article 100 further comprises an electronic system 22, which in turn comprises at least one type of sensor 32, an electric power source 34 and a transmitter 36, for transmitting measured data related to the absorbent article and/or to ambient conditions in close proximity to a wearer.

Alternatively, the re-usable part 20 may comprise a garment 50 to be worn by a user forming at least a portion of the chassis 11, whereas the disposable part 10 comprises the remaining portions of the chassis 11 and the absorbent core 13.

In an embodiment, some of the components of the garment 50 may be incorporated as a part of the holding means 16, 26 configured to attach the disposable part 10 to the re-usable part 20. By way of example, commonly known push-buttons at the crotch of a body suit may be used not only for closing said garment, but may be also used to attach the disposable 10 part to the re-usable part 20.

In this exemplary embodiment, the re-usable part 20 especially comprises a "smart-textile" garment, for example a "smart-textile" baby body suit or romper suit, in which the electronic system 22 or at least one element of the electronic system 22, is incorporated. Additionally or alternatively, some of the components of the garment may be incorporated into the electronic system 22 enabling connection among its elements. For example, push-buttons commonly placed at the crotch of the body suit 50 may also be adapted to connect the at least one sensor, placed e.g. in the disposable part 10, to the rest of the elements of the electronic system 22, which in turn are comprised into the "smart textile" of the body suit 50 (or in the re-usable part 20). By way of further example, the push-buttons of the re-usable garment 50 may be adapted to connect printed tracks of a wetness sensor 32 incorporated into the chassis 11 of the disposable part 10.

In a second aspect, the present invention provides a re-usable part 20 of an absorbent article configured to removably hold a disposable part 100 of the absorbent article, wherein the re-usable part 20 of an absorbent article 100 comprises an electronic system 22, or at least one element of the electronic system, the electronic system being configured to measure data related to the absorbent article and/or to ambient conditions in close proximity to a wearer. Embodiments of the re-usable part are depicted in the context of Figs. 2 to 7.

Preferably, said re-usable part 20 is configured to extend along at least a width of the disposable part 10 and along at least a portion of a length of the disposable part 10. In this manner, when the absorbent article is put on a wearer, the re-usable part 20 extends around the entire waist of the disposable part 10 of the absorbent article, securely supporting said disposable part 10.

In an embodiment, the re-usable part 20 is preferably washable; additionally or as an alternative, it is made of textile material. In addition or as a further alternative, at least portions of said re-usable part 20 have elastic properties. Moreover, the re-usable part 20 is configured to make use of the technology known as "smart textiles", "smart garments", or "flexible electronics", in a manner that it may easily integrate the electronic system 22. Advantageously, it is worth noticing that the re-usable part 20 herewith disclosed is less prone to be contaminated by body exudates and hence there is no need to be washed often, reducing its environmental footprint and enhancing its lifetime.

Preferably, the electronic system 22 comprises at least one type of sensor 32 and a transmitter 36, as depicted in Fig. 3, for transmitting the measured data related to the absorbent article and/or to ambient conditions in close proximity to a wearer. Additionally, the electronic system 22 preferably comprises an energy harvester. In addition to this, the at least one type of sensor 32 is a humidity sensor and/or a wetness sensor and/or a sensor for measuring ambient parameters and/or a sensor for measuring body parameters a sensor for measuring parameters of the absorbent article and/or any combination thereof.

The mentioned parameters may comprise at least one of humidity, wetness, gas concentration, acceleration, body orientation (detected by means of a gyroscope), body temperature, heart rate, blood pressure, ambient temperature, air pressure, electric field strength, magnetic field strength, volume of sound, radiation dose, or any combination thereof.

The re-usable part 20 may further comprise a fastening system 24 for the absorbent article. Alternatively, the re-usable part 20 may comprise some elements of the fastening system 24 configured to work in in combination with complementary elements 14 when attached to a disposable part 10. Preferably, the re-usable part 20 may further comprise at least a part of a holding means 26 configured to attach said re-usable part 20 to a disposable part 10 to form an absorbent article 100 in accordance with the first aspect of the invention. Additionally or alternatively, holding means 26 may be present only on the disposable part 20. Thus, advantageously, the re-usable part 20 herein disclosed may be used in combination with any absorbent article, e.g. a baby diaper or an adult diaper, existing in the market as long as such diaper fits the holding means provided by the re-usable part 20 as well as its dimensions. In this manner, the electronic system 22 incorporated into said re-usable part 20, and thereby its functionality, may still be employed irrespective of using the specific disposable part 10 as encompassed in the first aspect of the invention.

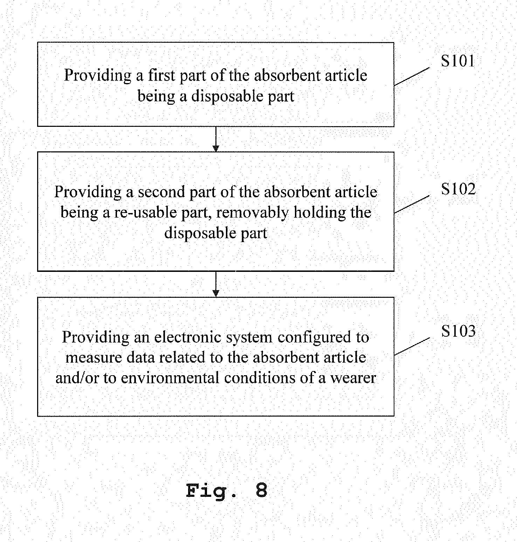

In still a further aspect, the present invention provides a method for attaching parts of an absorbent article. A preferred embodiment for the method is depicted in Fig. 8. The inventive method comprises the steps of: providing S101 a first part of the absorbent article being a disposable part; providing S102 a second part of the absorbent article being a re-usable part, wherein the re-usable part is configured to removably hold the disposable part.

The method further comprises providing S103 an electronic system, whereby the electronic system is configured to measure data related to the absorbent article and/or to ambient conditions in close proximity to a wearer. Said electronic system may comprise at least one type of sensor and a transmitter, for transmitting measured data related to the absorbent article and/or to ambient conditions in close proximity to a wearer. In addition to this, the electronic system may further comprise an energy harvester. The at least one type of sensor may be a humidity sensor and/or a wetness sensor and/or a sensor for measuring ambient parameters and/or a sensor for measuring body parameters and/or a sensor for measuring parameters of the absorbent article and/or any combination thereof.

It is supposed that the present invention is not restricted to any form of realization described previously and that some modifications can be added to the presented examples without reappraisal of the appended claims.

## Claims

1. An absorbent article (100) comprising
a first part being a disposable part (10), and
a second part being a re-usable part (20),
wherein the re-usable part (20) is configured to removably hold the disposable part (10),
**characterized in that**
the absorbent article (100) comprises an electronic system (22) configured to measure data related to a wearer of the absorbent article, and/or to the absorbent article and/or to ambient conditions in close proximity to the wearer of the absorbent article.

2. The absorbent article (100) according to claim 1,
wherein the electronic system (22) comprises at least one type of sensor (32) and a transmitter (36), for transmitting measured data related to the absorbent article and/or to ambient conditions in close proximity to a wearer.

3. The absorbent article (100) according to claims 1 or 2,
wherein the electronic system (22) further comprises an energy harvester (34).

4. The absorbent article (100) according to any of claims 2 or 3,
wherein the at least one type of sensor (32) is a humidity sensor and/or a wetness sensor and/or a sensor for measuring ambient parameters and/or a sensor for measuring body parameters and/or a sensor for measuring parameters of the absorbent article and/or any combination thereof.

5. The absorbent article (100) according to any of the preceding claims,
wherein the electronic system (22) or at least one element of the electronic system (22) is
i) included in a device separated from the re-usable part (20) and the disposable part (10), in a manner that it is mechanically connected to the absorbent article, and/or
ii) integrated into the re-usable part (20), and/or
iii) integrated into the disposable part (10), and/or
any combination thereof.

6. The absorbent article (100) according to any of the preceding claims,
wherein the re-usable part (20) is configured to extend along at least a portion of a width of the disposable part (10) and to extend along at least a portion of a length of the disposable part (10), and/or
wherein the disposable part (10) comprises at least an absorbent core (13).

7. The absorbent article (100) according to any of the preceding claims,
wherein the re-usable part (20) comprises an elongated part in the form of a belt, whereas the disposable part (10) comprises a chassis (11) of the absorbent article (100).

8. The absorbent article (100) according to any of the preceding claims,
wherein the re-usable part (20) comprises at least a portion of a chassis (11) of the absorbent article (100) whereas the disposable part (10) comprises remaining portions of the chassis (11).

9. The absorbent article (100) according to any of the preceding claims
wherein the re-usable part (20) comprises a garment (50) to be worn by a user forming at least a portion of a chassis of the absorbent article, whereas the disposable part (10) comprises remaining portions of the chassis (11).

10. The absorbent article (100) according to any of the preceding claims,
wherein the re-usable part (20) comprises a fastening system (14, 24) of the absorbent article (100), or
wherein the disposable part (10) comprises a fastening system (14, 24) of the absorbent article (100), or
wherein the re-usable part (20) comprises elements of the fastening system of the absorbent article (100) and the disposable part (10) comprises complementary elements of the fastening system (14, 24).

11. The absorbent article (100) according to any of the preceding claims,
wherein the absorbent article further comprises at least one holding means (16, 26) configured to attach the re-usable part (20) to the disposable part (10), and
wherein the holding means (16, 26) is present on the disposable part (10) or on the re-usable part (20) or on the disposable part (10) and the re-usable part (20).

12. A re-usable part (20) of an absorbent article configured to removably hold a disposable part (10) of the absorbent article,
**characterized in that**
the re-usable part (20) of the absorbent article comprises an electronic system (22), or at least one element of the electronic system (22), the electronic system (22) being configured to measure data related to a wearer of the absorbent article, and/or the absorbent article, and/or to ambient conditions in close proximity to the wearer of the absorbent article.

13. The re-usable part (20) according to claim 12,
wherein the electronic system (22) comprises at least one type of sensor (32) and a transmitter (36) for transmitting the measured data related to the wearer of the absorbent article, and/or the absorbent article, and/or to ambient conditions in close proximity to the wearer of the absorbent article, , and
wherein the at least one type of sensor (32) is a humidity sensor and/or a wetness sensor and/or a sensor for measuring ambient parameters and/or a sensor for measuring body parameters and/or a sensor for measuring parameters of the absorbent article and/or any combination thereof.

14. The re-usable part (20) according to claims 13,
wherein the electronic system (22) further comprises an energy harvester (34).

15. The re-usable part (20) according to any of the claims 12 to 14,
wherein said re-usable part (20) is configured to extend along at least a portion of a width of the disposable part (10) and along at least a portion of a length of the disposable part (10).

16. The re-usable part (20) according to any of claims 12 to 15,
wherein said re-usable part (20) is washable and/or is made of textile material, and/or
wherein at least portions of said re-usable part (20) has elastic properties.

17. A method for holding parts of an absorbent article comprising the steps of
providing a first part of the absorbent article being a disposable part (10); and
providing a second part of the absorbent article being a re-usable part (20),
wherein the re-usable part (20) is configured to removably hold the disposable part (10);
**characterized by**
providing an electronic system (22), and
wherein the electronic system (22) is configured to measure data related to a wearer of the absorbent article, and/or to the absorbent article, and/or to ambient conditions in close proximity to the wearer of the absorbent article.
